# EUROPEAN PATENT APPLICATION

(11) **EP 2 020 442 A1**
(43) Date of publication of application: **04.02.2009**
(21) Application number: 07015280.6
(22) Date of filing: 03.08.2007
(51) Int. Cl.: C12N 15/67

(54) **Enhancement of protein production in eukaryotic cells**

(71) Applicant: Max-Planck-Gesellschaft zur Förderung der Wissenschaften e.V., 80539 München (DE)
(72) Inventor: Schweiger, Susann, Dundee DD5 3BB (GB); Schneider, Rainer, 6300 Wörgl (AT); Aranda-Orgillés, Beatriz, 10435 Berlin (DE); Roepcke, Stefan, 78462 Konstanz (DE); Krause, Sven, 37079 Göttingen (DE)
(74) Representative: Arth, Hans-Lothar

(57) **Abstract**

The present invention relates to the increased expression of polypeptides in eukaryotic cells. A method for increased production is described using an expression vector comprising a polynucleotide encoding a desired polypeptide and a G quartet like motif sequence.

## Description

The present invention relates to the increased expression of polypeptides in eukaryotic cells. A method for increased production is described using an expression vector comprising a polynucleotide encoding a desired polypeptide and a G quartet like motif sequence.

### Background of the invention

The production of eukaryote proteins in cell systems is an important issue for a wide range of applications in modern biotechnology. Applications range from research into the biological function of proteins to their production as biopharmaceuticals and diagnostic reagents and the development of transgenic animals and plants. However importantly, most of these applications require correct post-translational modification. Production of eukaryote proteins in prokaryotes is limited by the lack of post-translational processing, including appropriate protein folding and secondary modifications such as glycosylation and some phosphorylations. Also, the absence of most cell organelles prohibits the functional analysis of eukaryotic proteins in prokaryotes. Thus many applications, including gene therapy, the fabrication of recombinant protein therapeutics and diagnostics and the production of proteins as well as the production of transgenic plants and animals for in vivo and in vitro studies, depend on proteins produced in eukaryotic cells. Unfortunately, the relatively low productivity of most eukaryotic cell systems is the most significant hindrance.

Efforts have been undertaken during the last few years to improve productivity of eukaryotic protein expression systems. The site of integration of a plasmid into the genome of an acceptor cell line has a major impact on its transcription, for example, integration into heterochromatin results in little or no expression. Several strategies have been developed to overcome this problem. Targeted integration via homologous recombination is one possibility and enzymes with recombinase activity, such as bacteriophage P1 Cre recombinase, lambda phage integrase or yeast Flp recombinase, can be used to enhance the probability of targeted integration. Also due to the strong influence of splicing on translation most modern expression vectors include an intron between the promoter and the cDNA coding sequence. Additionally, engineering of rare tRNA codons into more frequently used tRNA codons encoding identical amino acids, can be used to convert a gene expressed at low levels into a high-expressing gene. In another strategy host cells have been genetically modified into higher efficiency producers by integrating genes into their genome that make them resistant to influences that reduce viability or growth-promoting proto-oncogenes, cell cycle control genes, growth factors and anti-apoptotic genes.

The present invention surprisingly found that protein production is increased by polypeptide translation from an mRNA carrying G-quartet like sequences. It is the object of the present invention to provide a method and expression vector that optimizes the productivity of eukaryotic expression systems at the translation level.

The object of the present invention is solved by the teaching of the independent claims. Further advantageous features, aspects and details of the invention are evident from the dependent claims, the description, and the examples of the present application.

### Description of the invention

The present inventors have identified a cytoskeleton-associated ribosomal complex that includes active polyribosomes and components of the translation-controlling mTOR (mammalian target of rapamycin) pathway, such as the MID1 and α4 proteins, which is present in all cell types tested, including yeast and insect cells. mRNA associated to this complex is stabilized, thus significantly increasing their translation. Furthermore, the present inventors found that mRNA containing G-quartet like RNA motifs having a consensus sequence of WGG-N(1-4)-WGG-N(1-4)-WGG-N(1-4)-WGG has a high affinity to the described ribosome/mTOR complex.

G-quartet like RNA motifs have been described previously, however the biological function of these structures is still under investigation. They have been variously associated with mRNA turnover and HIV packaging, as well as being proposed to have a regulatory role in cell metabolism.

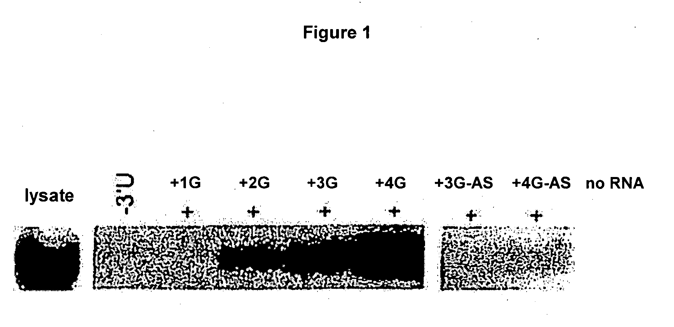

The present inventors have demonstrated that G-quartet like motifs having a consensus, sequence of WGG-N(1-4)-WGG-N(1-4)-WGG-N(1-4)-WGG mediate the binding of mRNAs carrying the G-quartet like sequence to the ribosome complex. Further the present inventors have demonstrated that binding is increased in a linear additive manner by the presence of G-quartet like motif sequences. As described in Example 1 and shown in Figure 1 as the number of G-quartet like motif sequences in an mRNA was increased up to four G-quartet like motifs, the amount of the protein production from the mRNA also increased.

The position of the G-quartet like motif(s) within the respective mRNA does not seem to play a role, meaning that the G-quartet like motif sequence can be located inside or outside (3' or 5') of an open reading frame. When the positioning of essential sequence G-quartet like sequence motifs is outside of the open reading frame, this method will not influence the protein sequence or post-translational modifications, guaranteeing unaltered quality of the produced proteins.

Without wishing to be bound by any particular theory the inventors propose that the possible molecular basis of the increase in protein production is both an increase of mRNA stability mediated by proteins interacting with the complex and an induction of translation via the mTOR signalling cascade.

We describe herein a strategy to enhance protein production by increased translation from an mRNA carrying G-quartet like sequences 3' or 5' of or within an open reading frame in cell lines and transgenic plants and animals. Furthermore, protein amounts produced in the host cell lines or transgenic plants or animals can be titered upon the number of G-quartets, preferably up to five incorporated in the vector.

Preferably the G-quartet motif encodes the following sequence:

NGGN₍₂₋₅₎GGN₍₂₋₅₎GGN₍₂₋₅₎GG,

wherein N is any nucleotide.

Further preferably the G quartet motif sequence encodes an RNA consensus sequence of:

WGGN₍₁₋₄₎WGGN₍₁₋₄₎WGGN₍₁₋₄₎WGG

wherein N is any nucleotide and W is A or T.

Preferably, if incorporated 3' of an open reading-frame, the RNA motif enhances protein production 2-10 fold. Further preferably co-transfection with a plasmid expressing the MID1 or α4 gene additionally increases protein production another 5-fold.

Also in the present invention the protein production increases according to the increasing number of G-quartets present and can be regulated by the number of G-quartets incorporated in the vector. Thus the amount of polypeptide produced is increased as the number of G quartet motif sequences is increased. For example, a larger amount of polypeptide is produced when four G quartet motif sequences are used that when only one G quartet motif sequence is used. Preferably up to five G-quartet like motifs shall be used in the same vector.

As used herein "increased production of a polypeptide" is defined as that the amount of a polypeptide produced by a host cell in the presence of the G quartet motif sequence is greater than the amount of the polypeptide produced in the absence of the G quartet motif sequence.

As used herein "polypeptide" is defined as a polymer of amino acids joined by peptide bonds. The polypeptide is encoded by a corresponding polynucleotide sequence.

As used herein "polynucleotide sequence" is defined as a DNA sequence capable of encoding a polypeptide.

As used herein "protein" is defined as a macromolecule comprising one or more polypeptide chains. A protein may also comprise non-peptide components, such as carbohydrate groups.

As used herein "expression vector" is defined as a nucleic acid molecule encoding a polynucleotide sequence that is expressed in a host cell. The expression vector can also comprise regulatory sequences, termination sequences and sequence encoding a selection factor(s). The expression vector may be, for example, a plasmid or a viral or retroviral vector, such as the pLenti6-V5, the pLenti6/V5-DEST Gateway™, the pAd/PL-DEST™ the pAd/CMV/V5-DEST™, the BD BaculoGoldTM, the AcNPV, the pCMV/Blue/OuabainR, the pCMV/OuabainR, the pRK-5, the pAPtag-4, the Gateway™ pCMV•SPORT6, the pcDNA™3.1, the pcDNA™4, the pcDNA™5, the pcDNA™6, the pCEP4, the pCMV, the pDisplay™, the pEF, the pEF1, the pEF4/, the pEF6, the Plasmid pCMV•SPORT, the pREP4, the pSecTag2, the pTracer™-EF, the pUB6, the pVAX1, the pVP22, the pZeoSV2, the pBud, the pIRES, the pCI, the pSI, the pTARGET™, the pFLAG®-MAC, the p3XFLAG-CMV™, the pBICEP-CMV™, the pcDNA, the pCMV-FLAG®, the pFLAG®-CMV, the pRc, the pGS, the pUSEamp, the pGL, the pRL, the phMGFP, the pEGFP, the pGFP, the pYFP, the pRFP, the pDSred, the pSV-β-Gal, the pCAT®3, the pHTS-MCS, the pcherry, the pHT3, the pStec, the FMHGW, the rtTA, the pHR'CMV, the pSNVRU5Iuc, the pSNVluc, the pTRL2, the pCALwL, the tTS, the psiRNA™, the pBROAD, the pWHERE, the pMONO, the pSELECT, the pVITRO, the pVIVO, the pFUSE-Fc and others.

As used herein "host cell" is defined as a cell capable of producing a polypeptide encoded by a polynucleotide sequence carried on an expression vector. The host cell may be, for example, a yeast cell, an insect cell, or a mammalian cell. Examples of specific host cells include the following: any insect cell line of interest, any plant cell of interest, any mammalian cells of interest or yeast or any transgenic animal cells of interest. For example, Table 1 provides a listing of possible mammalian host cells that could be used with an expression vector containing at least one G quartet motif sequence according to the present invention. However the listing is not exhaustive and the expression vector containing at least one G quartet motif sequence according to the present invention could be used in a mammalian host cell that is not listed in Table 1.

**Table 1. Mammalian host cells**

| Cell line | Cell type | Species |
|---|---|---|
| 143B | Osteosarcoma | Human |
| 15P-1 | Sertoli (Testis) | Mouse |
| 16HBE | Epithelial airway | Human |
| 293, 293H, 293F | Kidney | Human |
| 293T | Embryonic kidney | Human |
| 3T3-F442A | Adipocytes | Mouse |
| A10 | Smooth muscle | Rat |
| A20.J; M12 | Lymphoid | Mouse B cell lymphoma |
| A498; UMRC 6 | Kidney carcinoma | Human |
| A549 | Lung | Human carcinoma |
| AKR | Spleen | Mouse |
| AR4-2J | Pancreas | Rat |
| AS52; CHO-DXB11 | Ovary | Hamster |
| AtT-20 | Pituitary | Mouse |
| B16 | Melanoma | Mouse |
| BEAS-2B | Bronchial epithelial cells | Human |
| BL3.1 | Lymphoid | Bovine leukemia |
| Blastoderm cells | | Chicken |
| C127 | Breast Cancer | Mouse |
| C2C12 | Myoblast | Mouse |
| C2C12 | Muscle | Cow |
| C-33A | Cervical Cancer | Human |
| C6 | Glioma | Rat |
| Capan-2 | Pancreas cancer | Human |
| CHO | Ovary (epithelial like) | Chinese Hamster |
| CHO-K1 | Ovary | Hamster |
| COLO 320 HSR; SW40; 3SW480; Caco-2 | Colon | Human cancer |
| COS1, COS-7 | Kidney | Green Monkey |
| CT60 | Ovary | Hamster |
| D3 | Stem cells | Mouse |
| Dede | Lung | Hamster |
| F11 | Mouse neuroblastoma + Rat DRG neurons | Mouse/Rat |
| FLK | Kidney | Sheep |
| FTO-2B | Liver cancer | Rat |
| GD25-β1 | Embryonal fibroblast | Mouse |
| GH3 | Pituitary tumor | Rat |
| H187; NCl-H146 | Lung | Human small cell carcinoma |
| HBL-100 | Transformed Breast | Human |
| HCT116 | Colon | Human cancer |
| HEK293 | Embryonic kidney | Human |
| HeLa | Cervical Cancer | Human |
| Hep 3B | Liver cancer | Human |
| Hep2 | Laryngeal Epithelium | Human |
| HepG2 | Hepatoma | Human |
| Hs 578T; T-47D; MDA-MB-231; ZR-75-1 | Breast Cancer | Human |
| HT-29 | Colon | Human cancer |
| Huh7 | Liver cancer | Human |
| Isikawa | Endometrial cancer | Human |
| Jurkat | Lymphoid | Human lymphoma |
| KG-1; KG1a; K562 | leukemia | Human |
| L | Connective Tissue | Mouse |
| L6E9 | Muscle | Rat |
| LLC | Lung | Mouse carcinoma |
| LLC-PK | Kidney | Pig |
| LNCaP; PC-3 | Prostate | Human |
| MCF-7 | Breast Adenocarcinoma | Human |
| MCF7 | Breast Cancer | Human |
| MDCK | Kidney | Canine |
| MeWo | Melanoma | Human |
| MOLT-4 | Lymphoid | Human leukemia |
| MRC-5 | Lung | Human |
| MSN610.2; Morris 7777; H35; McA-RH7777 | Liver cancer | Rat |
| Mv 1 Lu | Lung Fibroblasts | Mink |
| N2A | Neuroblastoma | Mouse |
| NCI-H23 | Lung | Human cancer |
| Neuro-2a | Neuroblastoma | Mouse |
| NG 108-15 | Neurablastoma-glioma fusion | Mouse-Rat |
| NIH 3T3; TA1; Y-2; NIH 3T6; 10T1/2 | Fibroblasts | Mouse |
| NI H-3T3 | Fibroblasts | Mouse |
| NMU | Breast Cancer | Rat |
| P19; F9 | Embryonic carcinoma | Mouse |
| P388D1 | Lymphoid | Mouse lymphoma |
| PC 12 | Pheochromocytoma | Rat |
| Primary chondrocytes | | Chicken |
| Primary Endothelial | Endothelial | Rabbit |
| Primary fetal astrocyte | Astrocyte | Rat |
| Primary fetal islet cells | Pancreas islet | Rat |
| Primary Fibroblast | Fibroblasts | Human |
| Primary fibroblasts | | Chicken |
| Primary hematopoetic CD34+ | Stem cells | Human |
| Primary hepatocytes | | Chicken |
| Primary HUAEC | Endothelial | Human |
| Primary HUVEC | Endothelial | Human |
| Primary keratinocytes | Skin | Human |
| Primary Liver | Liver | Dog |
| Primary Liver | Liver | Mouse |
| Primary Liver | Liver | Rat |
| Primary Ovary | Ovary | Mouse |
| Primary Pituitary | Pituitary | Rat |
| Primary; G8 | Muscle | Mouse |
| Primary; J774.16; RAW 264.7 | Monocytes | Mouse macrophage |
| R1610 | Lung | Hamster |
| SK-BR-3 | Breast Cancer | Human |
| SK-N-MC | Neuroblastoma | Human |
| SK-N-SH | Neuroblastoma | Human |
| SKOV3 | Ovary cancer | Human |
| SK-UT-1 | Uterus | Human |
| Sperm | Sperm | Mouse |
| SW13 | Adrenal carcinoma | Human |
| Swiss 3T3 | Fibroblasts | Mouse |
| T24 | Bladder carcinoma | Human |
| T84 | Colon | Human cancer |
| THP-1 | Macrophages/ | Human monocyte |
| Trachea smooth muscle | Muscle | Cow |
| U-2 OS | Bone sarcoma | Human |
| U373 | Glioblatoma | Human |
| U-87; A-172 | Glioma | Human |
| U937 | Lymphoid | Human lymphoma |
| WI-38 | Lung | Human |

As used herein a "host" is an organism capable of producing a polypeptide encoded by a polynucleotide sequence carried on an expression vector. The host may be, for example, a transgenic animal

Thus the present invention is directed to a method for increasing production of a polypeptide in a eukaryote host cell comprising transforming the eukaryote host cell with an expression vector comprising a polynucleotide sequence that encodes an open reading frame for the polypeptide and at least one and preferably up to five G quartet like motif sequences, culturing the transformed cells under suitable conditions and isolating the expressed polypeptide.

General methods for constructing expression vectors, transforming host cells, culturing host cells and isolating an expressed polypeptide are well known in the art. For example, the commercial products and accompanying protocols available from any of Invitrogen Corporation, InvivoGen, Promega Corporation, or Sigma-Aldrich Co. are suitable to carry out the present invention.

In the method of the present invention the amount of a polypeptide produced in the presence of a G quartet motif sequence is greater than the amount of polypeptide produced in the absence of a G quartet motif sequence.

Preferably the number of G quartet motif sequences is between 1 and 5, more preferably between 3 and 4.

In the method of the present invention the amount of the polypeptide produced is increased as the number of G quartet motif sequences is increased.

The method of the present invention is suitable for use in an in vitro eukaryote expression system. The method of the present invention is also suitable for use in an in vivo eukaryote expression system.

Preferably in the expression vector the G quartet motif sequence is situated 3' of the polypeptide open reading frame. When the G quartet motif sequence is incorporated 3' of the open reading-frame preferably protein production is increased 2-10 fold.

Alternatively in the expression vector the G quartet motif sequence is situated within the polypeptide opens reading frame.

Preferably the G-quartet motif encodes the following sequence:

NGGN₍₂₋₅₎GGN₍₂₋₅₎GGN₍₂₋₅₎GG,

wherein N is any nucleotide.

Further preferably the G quartet motif sequence encodes an RNA consensus sequence of:

WGGN₍₁₋₄)WGGN₍₁₋₄₎WGGN₍₁₋₄₎WGG

wherein N is any nucleotide and W is A or T.

Preferably the polypeptide is a human polypeptide. Alternatively the polypeptide is an animal polypeptide. Further preferably the polypeptide is a recombinant polypeptide. Further preferably the polypeptide is a plant polypeptide.

Additionally the eukaryote cell can be further transformed with an expression vector encoding the MID1 protein. Preferably when the host cell is co-transfected with a plasmid expressing the MID1 gene protein production is increased 5-fold. The host cell can also be further transformed with an expression vector encoding the α4 protein.

Preferably the host cell is any insect cell of interest or any plant cell of interest or any mammalian cell of interest or yeast or any transgenic animal cell of interest.

The present invention is also directed to the use of a G quartet motif sequence to increase production of a polypeptide in a eukaryote host cell, wherein the eukaryote host cell is transformed with an expression vector comprising a polynucleotide sequence that encodes an open reading frame for the polypeptide and at least one G quartet motif sequence.

In the use of the present invention the amount of a polypeptide produced in the presence of a G quartet motif sequence is greater than the amount of polypeptide produced in the absence of a G quartet motif sequence.

Preferably the number of G quartet motif sequences is between 1 and 5, more preferably between 3 and 4.

In the use of the present invention the amount of the polypeptide produced is increased as the number of G quartet motif sequences is increased.

The G quartet motif sequence of the present invention is suitable for use in an in vitro eukaryote expression system. The G quartet motif sequence of the present invention is also suitable for use in an in vivo eukaryote expression system.

Preferably in the expression vector the G quartet motif sequence is situated 3' of the polypeptide open reading frame. When the G quartet motif sequence is incorporated 3' of the open reading-frame preferably protein production is increased 2-10 fold.

Alternatively in the expression vector the G quartet motif sequence is situated within the polypeptide open reading frame.

Preferably the G-quartet motif encodes the following sequence:

NGGN₍₂₋₅₎GGN₍₂₋₅₎GGN₍₂₋₅₎GG,

wherein N is any nucleotide.

Further preferably the G quartet motif sequence encodes an RNA consensus sequence of:

WGGN₍₁₋₄₎WGGN₍₁₋₄₎WGGN₍₁₋₄₎WGG

wherein N is any nucleotide and W is A or T.

Preferably the polypeptide is a human polypeptide. Alternatively the polypeptide is an animal polypeptide. Further preferably the polypeptide is a recombinant polypeptide. Further preferably the polypeptide is a plant polypeptide.

Additionally the eukaryote cell can be further transformed with an expression vector encoding the MID1 protein. Preferably when the host cell is co-transfected with a plasmid expressing the MID1 gene protein production is increased 5-fold. The host cell can also be further transformed with an expression vector encoding the α4 protein.

Preferably the host cell is any insect cell of interest or any plant cell of interest or any mammalian cell of interest or yeast or any transgenic animal cell of interest.

The present invention is further directed to an expression vector comprising a polynucleotide encoding an open reading frame for a polypeptide and at least one G quartet motif sequence, wherein the G quartet motif sequence increases production of the polypeptide in a eukaryote host cell.

In a host cell transformed with the expression vector of the present invention the amount of a polypeptide produced in the presence of a G quartet motif sequence is greater than the amount of polypeptide produced in the absence of a G quartet motif sequence.

Preferably the number of G quartet motif sequences is between 1 and 5, more preferably between 3 and 4.

In a host cell transformed with the expression vector of the present invention the amount of the polypeptide produced is increased as the number of G quartet motif sequences is increased.

The expression vector of the present invention is suitable for use in an in vitro eukaryote expression system. The expression vector of the present invention is also suitable for use in an in vivo eukaryote expression system.

Preferably in the expression vector the G quartet motif sequence is situated 3' of the polypeptide open reading frame. When the G quartet motif sequence is incorporated 3' of the open reading-frame preferably protein production is increased 2-10 fold.

Alternatively in the expression vector the G quartet motif sequence is situated within the polypeptide open reading frame.

Preferably the G-quartet motif encodes the following sequence:

NGGN₍₂₋₅₎GGN₍₂₋₅₎GGN₍₂₋₅₎GG,

wherein N is any nucleotide.

Further preferably the G quartet motif sequence encodes an RNA consensus sequence of:

WGGN₍₁₋₄₎WGGN₍₁₋₄₎WGGN₍₁₋₄₎WGG

wherein N is any nucleotide and W is A or T.

Preferably the polypeptide is a human polypeptide. Alternatively the polypeptide is an animal polypeptide. Further preferably the polypeptide is a recombinant polypeptide. Further preferably the polypeptide is a plant polypeptide.

Additionally the expression vector further comprises polynucleotide sequence encoding the MID1 protein. Preferably when a host cell is co-transfected with an expression vector expressing the MID1 gene protein production is increased 5-fold. The expression vector can also further comprise polynucleotide sequence encoding the α4 protein.

Preferably the host cell is any insect cell of interest or any plant cell of interest or any mammalian cell of interest or yeast or any transgenic animal cell of interest.

### Description of the figures:

Figure 1: EFNB1 mRNAs either lacking the entire 3'UTR (-3'U) or including one G-quartet (+1G), two G-quartets (+2G), three G-quartets (+3G) or four G-quartets (+4G), with antisense transcripts of 3 G-quartet (+3G-AS) and four G-quartet (+4GAS) mRNAs used as background controls. G-quartets were amplified and *in vitro* transcribed using biotinylated ribonucleotides. Elution fractions of RNA-protein pull-down assays in FLAG-MID1 over-expressing cells were analysed for the presence of FLAG-MID1 with an anti-FLAG antibody.

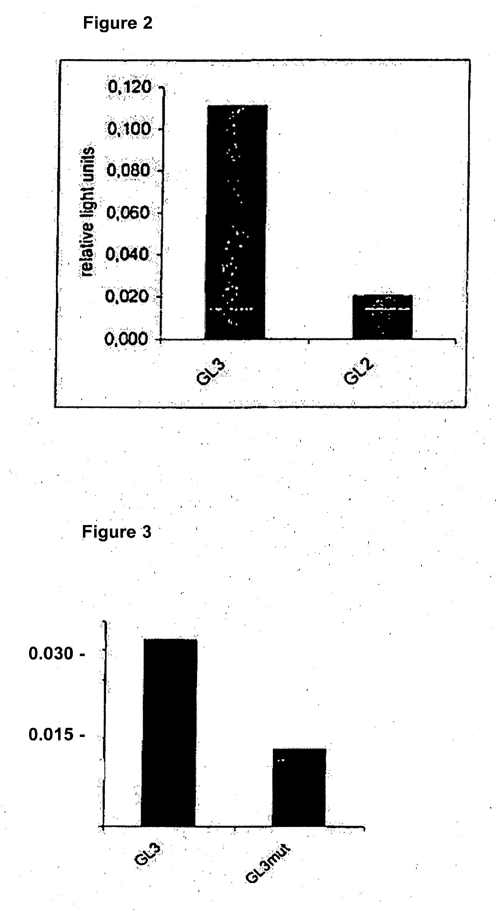

Figure 2: pGL3 had 6 times higher luciferase activity from the firefly luciferase gene compared to pGL2 as measured in relative light units.
Figure 3: Basal activities of the wild-type pGL3 (GL3) and a pGL3 vector mutated at a core guanine of the predicted G-quartet (GL3mut) as measured in relative light units.

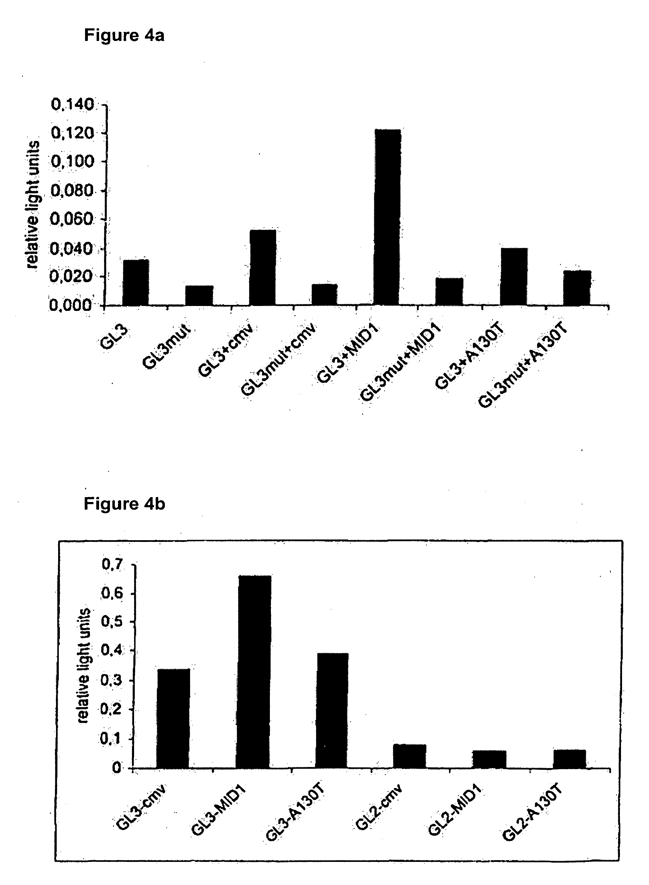

Figure 4: Induction of luciferase activity by co-transfection with wild-type MID1, but not with a mutated (A130T) form of MID1. Induction is seen of the pGL3 wild-type firefly luciferase gene (a, b) but not of a firefly luciferase gene with a mutated G-quartet (GL3mut) or the pGL2 firefly luciferase gene.

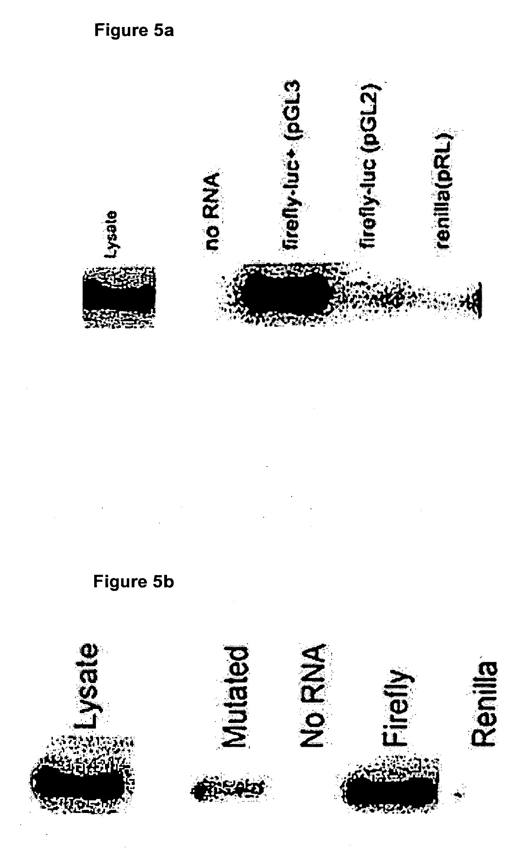

Figure 5: FLAG-MID1 was identified in the elution fraction of an RNA-protein pulldown assay performed with biotinylated firefly luciferase RNA in-vitro transcribed from the pGL3 vector (a, b), but not in an assay performed with firefly RNA transcribed from pGL2 (a), with renilla luciferase RNA (a, b) or in an experiment without RNA. Only little binding was observed with RNA transcribed from a pGL3 vector carrying a mutation in the G-quartet structure (b).

### EXAMPLES

### Example 1: G-quartet motifs bind to a microtubule-associated mRNP

MID1 and α4 are the core of a microtubule-associated mRNP that, in addition to active ribosomes, also assembles G-rich mRNA motifs. In an RNA-protein pull-down experiment, we found that G-quartet structures with the consensus sequence of WGG-N(1-4)-WGG-N(1-4)-WGG-N(1-4)-WGG have a particularly high affinity to the mRNP.

G-quartet motifs from different mRNAs (EFNBIB, EPBH2b, EFNB2a, EPBH3c) were amplified and *in vitro*-transcribed with biotinylated ribonucleotides. Transcripts were then immobilized on streptavidin-coated beads and loaded with lysates from MID1-FLAG over-expressing HeLa cells. Elution fractions were analysed on SDS gels using the respective antibodies detecting members of the MID1 protein complex and binding of mRNP to the G-quartet structures was demonstrated.

*G-quartet motifs bind to* a *microtubule-associated mRNP in an additive manner* A similar protein-RNA pull-down assay with biotinylated RNA *in vitro* transcribed from a plasmid containing the open reading frame and 3'UTR of ephrin B1 (EFNB1) showed an additive effect of multiple G-quartets on the binding affinity of the respective RNA to the mRNP.

We have used EFNB1 mRNAs with differing lengths, including only the open reading frame or the open reading frame and different parts of the 3'UTR with one, two, three or four G-quartets. Antisense transcripts of the two longest mRNAs were used as background controls. G-quartets were amplified and *in vitro* transcribed using biotinylated ribonucleotides. Elution fractions of RNA-protein pull-down experiments with HeLa cells over-expressing FLAG-tagged MID1 were subsequently analysed by Western blot analysis with an anti-FLAG antibody, and showed a linear increase of the binding affinity between mRNA and MID1 proportional to the number of G-quartet structures (see Figure 1).

### Example 2: Comparison of luciferase activity: pGL2 and pGL3

Luciferase assays showed a 6-fold higher basal activity of firefly luciferase activity from pGL3 vector compared to the pGL2 vector (see Figure 2). Ratios between firefly and renilla luciferase were measured in a dual luciferase vector system.

### Alignment between pGL2 and pGL3

Sequence alignments of pGL2 and pGL3 show identity of the two vectors over large parts of the sequence. Only a few base pair mismatches were detected within the open reading frame of the firefly luciferase. Among these, four base pair exchanges were found to be located in a G-quartet structure predicted for pGL3, thus eliminating the motif in pGL2.
GG**G**AT**A**CG**A**CAAGGATATGG pGL2
GG**T**AT**CA**G**G**CAAGGATATGG pGL3

### Example 3: Translation decrease in a pGL3 vector with mutated G-quartet

Next, we mutated one of the core guanines of the G-quartet predicted in the pGL3 firefly luciferase gene such that the amino acid sequence did not change, and checked luciferase activity of the mutated vector pGL3mut. Interestingly, basal activity of the mutated vector went down significantly (see Figure 3).

### Example 4: Increase of translation by co-transfection with MID1

Co-transfection of dual luciferase vectors (firefly, pGL and, as an internal control, renilla, pRL) with an expression vector containing the MID1 open reading frame showed an upregulation of firefly luciferase activity only when using the wild-type pGL3 vector, but not with the mutated pGL3 vector (Fig. 4a) or the pGL2 vector (Fig. 4b). Co-expression of the two luciferase vectors with a MID1-containing plasmid carrying a missense mutation that leads to dysfunction of the MID1 protein did not result in any induction of luciferase activity. Ratios between firefly and renilla luciferase were measured in a dual luciferase reporter system.

### Example 5: Binding of the pGL3 firefly luciferase mRNA to the MID1 protein complex

Specific binding of the MID1-protein complex to the wildtype pGL3 firefly luciferase mRNA (Fig. 5a and 5b) but not to the pGL2 firefly luciferase mRNA (Fig. 5a) nor to a pGL3 firefly luciferase mRNA carrying a mutation in the G-quartet (Fig. 5b) could be shown in an RNA-protein pulldown assay with biotin-streptavidin immobilized RNA and cell lysate from HeLa cells overexpressing FLAG-MID1. Elution fractions were analysed on a Western blot with an anti-FLAG antibody.

## Claims

1. A method for increasing production of a polypeptide in a eukaryote host cell comprising:
a) transforming the eukaryote host cell with an expression vector comprising a polynucleotide sequence that encodes an open reading frame for the polypeptide and at least one G quartet motif sequence;
b) culturing the transformed cells;
c) isolating the expressed polypeptide.

2. The method of claim 1, wherein the number of G quartet motif sequences is between 1 and 5.

3. The method of claim 1, wherein the amount of the polypeptide produced is increased as the number of G quartet motif sequences is increased

4. The method of claim 1, wherein in the expression vector the G quartet motif sequence is situated 3' of the polypeptide open reading frame.

5. The method of claim 1, wherein in the expression vector the G quartet motif sequence is situated within the polypeptide open reading frame.

6. The method of claim 1, wherein the G quartet motif sequence encodes an RNA consensus sequence of:
WGGN₍₁₋₄₎WGGN₍₁₋₄₎WGGN₍₁₋₄₎WGG.

7. The method of any one of claims 1 to 6, wherein the polypeptide is a human polypeptide.

8. The method of any one of claims 1 to 6, wherein the polypeptide is an animal polypeptide.

9. The method of any one of claims 1 to 6, wherein the polypeptide is a plant polypeptide.

10. The method of any one of the previous claims, wherein the polypeptide is a recombinant polypeptide.

11. The method of any one of the previous claims, wherein the eukaryote cell is further transformed with an expression vector encoding the MID1 protein.

12. The method of any one of the previous claims, wherein the eukaryote cell is further transformed with an expression vector encoding the α4 protein.

13. Use of a G quartet motif sequence to increase production of a polypeptide in a eukaryote host cell, wherein the eukaryote cell is transformed with an expression vector comprising a polynucleotide sequence that encodes an open reading frame for the polypeptide and at least one G quartet motif sequence.

14. An expression vector comprising a polynucleotide encoding an open reading frame for a polypeptide and at least one G quartet motif sequence, wherein the G quartet motif sequence increases production of the polypeptide in a eukaryote host cell.
